# EUROPEAN PATENT SPECIFICATION

(11) **EP 2 854 576 B1**
(45) Date of publication and mention of the grant of the patent: **23.12.2020**
(21) Application number: 13726206.9
(22) Date of filing: 30.05.2013
(51) Int. Cl.: A24D 1/20, A24D 1/22, A24C 5/18

(54) **BLENDED RODS FOR USE IN AEROSOL-GENERATING ARTICLES**
GEMISCHTE STÄBE ZUR VERWENDUNG BEI AEROSOLERZEUGUNGSARTIKELN
TIGES MÉLANGÉES DESTINÉES À ÊTRE UTILISÉES DANS DES ARTICLES DE GÉNÉRATION D'AÉROSOL

(30) Priority: 31.05.2012 EP 12170356
(43) Date of publication of application: 08.04.2015
(73) Proprietor: Philip Morris Products S.A., 2000 Neuchâtel (CH)
(72) Inventor: METRANGOLO, Alessandro, 2000 Neuchâtel (CH); GINDRAT, Pierre-Yves, 1907 Saxon (CH); FAULKNER, John, 2023 Gorgier (CH); SCHALLER, Jean-Pierre, 1202 Genève (CH); SCHNEIDER, Jean-Claude, 2012 Auvernier (CH)
(74) Representative: Bates, Alan Douglas Henry
(86) International application number: PCT/EP2013/061208
(87) International publication number: WO 2013/178766

(56) References cited:
- EP-A1- 0 503 767
- DE-A1- 19 854 009
- US-A- 3 472 236
- US-A- 4 168 712
- US-A- 4 807 809

## Description

The present specification relates to rods comprising two or more sheets of tobacco material gathered together to form a rod for use in aerosol-generating articles. The specification also relates to aerosol-generating articles comprising such rods, and methods for forming such rods.

Processes and apparatus for producing shreds, strands or strips of tobacco material are known in the art. Typically, the width of such shreds, strands and strips of tobacco material is about 3 mm or less.

For example, US-A-4,168,712 discloses cigarettes and filters for cigarettes, which are wholly or partly from sheet material such as reconstituted tobacco.

US-A-4,807,809 relates to the manufacture of rods for use in the manufacture of smoking articles, and in particular the manufacture of rods from a web of sheet-like material.

US-A-4,000,748 discloses a process and apparatus for shredding a sheet of reconstituted tobacco into strips and crimping the resultant strips in a substantially simultaneous operation. The sheet of tobacco material is moved between a pair of rotating and intermeshing stacks of disks which shred the sheet into a plurality of strips about 0.65 to 1.55 mm in width. The forward motion of the resultant strips is retarded by engagement with facing surfaces of neighbouring disks causing a buckling of the strips into a crimped configuration. The crimped strips are reported to provide an increase in fill value.

The formation of rods for aerosol-generating articles comprising crimped or uncrimped shreds of tobacco material suffers from a number of disadvantages including those discussed below.

Firstly, shredding tobacco material undesirably generates tobacco fines and other waste.

Secondly, rods comprising shreds of tobacco material exhibit 'loose ends'. That is, there is a loss of shreds of tobacco material from the ends of the rod. This is exacerbated by breakage of the shreds of tobacco material during rod formation. Loose ends are not only aesthetically undesirable, but can also disadvantageously lead to the need for more frequent cleaning of manufacturing equipment and aerosol-generating devices. The problem of loose ends is particularly exacerbated in aerosol-generating articles, because the rod length of aerosol-generating substrate tends to be low in comparison with conventional cigarettes, and therefore the proportion of substrate material that is in proximity to an end is greater.

Thirdly, rods comprising shreds of tobacco material exhibit high weight standard deviations. That is, rods of the same dimensions tend to be of inconsistent weight. This is due in part to the tendency of the rods to exhibit loose ends as mentioned above. The high weight standard deviation of rods comprising shreds of tobacco material leads to an undesirably high rejection rate of rods whose weight falls outside of a selected acceptance range. Furthermore, rods comprising shreds of tobacco material exhibit non-uniform densities. That is, the density along the rod length of the rod tends to be inconsistent. This is due to variations in the quantity of tobacco material at different locations along the rod, which results in 'voids', which are regions having reduced quantities of tobacco material, and 'pads', which are regions having increased levels of tobacco material. The non-uniform density of rods comprising shreds of tobacco material can undesirably affect the resistance to draw (RTD) of the rods. In addition, the non-uniform density of rods comprising shreds of tobacco material can lead to loose ends when a void is located at the end of the rod.

Loose ends, high weight standard deviations and non-uniform densities as exhibited by rods comprising shreds of tobacco material are particularly problematic and undesirable in rods of short rod length. Rods of short rod length are sometimes referred to as plugs.

There are many types of tobacco and many ways in which two tobacco types may differ. For example, different types of tobacco may derive from different tobacco plant varieties, or from different portions of a tobacco plant. Tobaccos may differ if they have been processed in different ways in order to vary certain characteristics. Tobaccos may differ if they comprise different additives, or different proportions of additives.

Aerosol-generating articles, such as traditional lit-end cigarettes, typically comprise blended tobacco. Blended tobacco typically comprises shreds and strips of many different types of tobacco leaf and homogenised tobacco. Properties of the cigarette such as flavour or perceived harshness may be controlled by blending many different types of tobacco.

EP-A1-2 062 484 discloses a process for forming smokeless tobacco articles for oral consumption. A sheet of reconstituted tobacco is gathered into a rod, wrapped, and cut into pieces suitable for oral consumption.

It would be desirable to provide rods comprising tobacco material for use in aerosol-generating articles.

A rod may be provided comprising a first sheet of tobacco material and a second sheet formed of a second tobacco material, wherein the second sheet is gathered together with the first sheet of tobacco material and circumscribed by a wrapper, wherein the first sheet of the first tobacco material and he second sheet of the second tobacco material have been disposed in overlapping relationship prior being gathered together, the first tobacco material and the second tobacco material being homogenised tobacco material, the second tobacco material being chemically different to the first tobacco material, wherein the first tobacco material comprises aerosol-forming compounds that form an aerosol at a lower temperature than aerosol-forming compounds of the second tobacco material.

A rod may be provided comprising at least two sheets of tobacco material gathered together and circumscribed by a wrapper, the rod including a first sheet of a first tobacco material and a second sheet of a second tobacco material, the second sheet of the second tobacco material being physically or chemically different to the first sheet of the first tobacco material.

By way of non-limiting examples, the two sheets may be physically different if they have different physical dimensions, different surface textures, different morphologies, different densities, or different porosities. Sheets may be textured by crimping and the crimping of one sheet may be of a different degree to the crimping of the other sheet. For example, one sheet may have a different depth of crimping compared to the other sheet or one sheet may have a different density of crimping, i.e. a different number of crimp lines per unit distance, compared to the other sheet. The skilled person will be aware of other physical differences that may characterise the two sheets of tobacco material.

By way of non-limiting examples, the two sheets may be chemically different if they contain different chemical constituents, for example if the two sheets contain different tobacco blends, or different types of aerosol former, or different types of plasticiser, or different flavourants, or different proportions of any element. For example, one sheet may comprise a different proportion of Virginia tobacco compared with the other sheet, or one sheet may comprise a different proportion of an aerosol former such as glycerine compared to the other sheet.

Once the first sheet and the second sheet have been gathered into a rod they may be described as gathered sheets. The gathered sheets of material preferably extend along substantially the entire rod length of the rod and across substantially the entire transverse cross-sectional area of the rod.

The tobacco material forming the at least two sheets is homogenised tobacco. The tobacco material may comprise an aerosol-former.

As used herein, the term 'rod' is used to denote a generally cylindrical element of substantially circular, oval or elliptical cross-section.

As used herein, the term 'sheet' denotes a laminar element having a width and length substantially greater than the thickness thereof. The width of a sheet is greater than 10 mm, preferably greater than 20 mm or 30 mm.

As used herein, the term 'rod length' denotes the dimension in the direction of the cylindrical axis of rods as described herein.

As used herein, the term 'homogenised tobacco' denotes a material formed by agglomerating particulate tobacco.

As used herein, the term 'gathered' denotes that the sheet of tobacco material is convoluted, folded, or otherwise compressed or constricted substantially transversely to the cylindrical axis of the rod.

As used herein, the terms 'upstream' and 'downstream' are used to describe the relative positions of components, or portions of components, of aerosol-generating articles comprising rods as described herein in relation to the direction of air drawn through the aerosol-generating articles during use thereof.

The inclusion of gathered sheets of homogenised tobacco material in rods as described herein advantageously significantly reduces the risk of loose ends compared to rods comprising shreds of tobacco material.

Rods comprising at least two sheets of tobacco material advantageously exhibit significantly lower weight standard deviations than rods comprising shreds of tobacco material. The weight of a rod of a particular rod length is determined by the density, width and thickness of the sheets of tobacco material that are gathered to form the rod. The weight of rods of a particular rod length can thus be regulated by controlling the density and dimensions of the individual sheets of tobacco material. This reduces inconsistencies in weight between rods of the same dimensions, and so results in lower rejection rate of rods whose weight falls outside of a selected acceptance range.

Rods comprising sheets of tobacco material as described herein advantageously exhibit more uniform densities than rods comprising shreds of tobacco material.

Tobacco sheet material such as homogenised tobacco sheet material may be produced using different types of tobacco. For example, tobacco sheet material may be formed using tobaccos from a number of different varieties of tobacco, or tobacco from different regions of the tobacco plant, such as leaves or stem. After processing, the sheet has consistent properties and a homogenised flavour. A single sheet of homogenised tobacco material may be produced to have a specific flavour. To produce a product having a different flavour, a different tobacco sheet material needs to be produced. Some flavours that are produced by blending a large number of different shredded tobaccos in a conventional cigarette may be difficult to replicate in a single homogenised tobacco sheet. For example, Virginia tobaccos and Burley tobaccos may need to be processed in different ways to optimise their individual flavours. It may not be possible to replicate a particular blend of Virginia and Burley tobaccos in a single sheet of homogenised tobacco material.

A rod may be provided comprising a sheet of a first tobacco material and a sheet of a second tobacco material that is chemically different from the first tobacco material. The sheets of tobacco material are gathered together to form the rod. By combining two different sheets of tobacco material in a single rod, new blends may be created that could not be produced by a single sheet of homogenised tobacco. This concept may be illustrated by the following non-limiting examples.

The first tobacco material may be dominated by Virginia tobacco and the second tobacco material may be dominated by Burley tobacco.

The first tobacco material may be dominated by Virginia tobacco and the second tobacco material may be dominated by Oriental tobacco.

The first tobacco material may be dominated by Virginia tobacco and the second tobacco material may comprise Perique tobacco.

The first tobacco material may be formed primarily from leaf tobacco and the second tobacco material may be derived primarily from stem tobacco.

The first tobacco material may be treated or aged or processed or cured in a different way from the second tobacco material such that it is chemically different.

Homogenised tobacco may comprise aerosol forming compounds such as glycerine and propylene glycol. This may be particularly advantageous when the homogenised tobacco is to be used as an aerosol-forming substrate of a heated aerosol-forming article.

The first tobacco material may comprise a first aerosol-forming compound and the second tobacco material may comprise a second aerosol-forming compound different to the first aerosol-forming compound. For example, it may be preferable to used glycerine as an aerosol-forming compound of a first sheet of tobacco material and triethylene glycol as an aerosol-forming compound of a second sheet of tobacco material.

A rod as described herein may be heated to evolve volatile compounds, which condense to form an inhalable aerosol. The temperature at which an aerosol forms and the rate at which the rod is consumed may depend to some degree on the nature of the aerosol-forming compounds present in the rod. It may be desirable to modify or improve a consumer experience by controlling the temperature and rate at which aerosol is evolved. It may be desirable that a rod comprises two different sheets of tobacco material that each provide a different aerosol evolution response to a heating profile.

The first tobacco material may comprise a different amount of aerosol-forming compound to the second tobacco material. For example, the first tobacco material may comprise 5% by weight glycerine, while the second tobacco material may comprise 10 % by weight glycerine.

The first tobacco material comprises aerosol-forming compounds that form an aerosol at a lower temperature than aerosol-forming compounds of the second tobacco material.

Homogenised tobacco materials may include various other additives such as humectants, plasticisers, flavourants, fillers, binders and solvents. Some additives may be more suitable for use with one type of tobacco material than another type of tobacco material.

The first tobacco material may comprise different additives to the second tobacco material.

The first tobacco material may comprise a different proportion of additives compared to the second tobacco material.

A rod may be provided comprising a first sheet of a first tobacco material and a second sheet of a second tobacco material that is physically or morphologically different from the first sheet. One of the sheets of tobacco material may be a textured sheet of material. Use of at least one textured sheet of material may advantageously facilitate gathering of the sheets to form a rod. Both or all of the sheets of tobacco material in a rod may be textured sheets of material.

As used herein, the term 'textured sheet' denotes a sheet that has been crimped, embossed, debossed, perforated or otherwise deformed. Crimping is a preferred form of texturing. Textured sheets of material may comprise a plurality of spaced-apart indentations, protrusions, perforations or a combination thereof.

The first sheet may be a textured sheet of tobacco material and the second sheet may be non-textured.

Both first and second sheets may be textured sheets of tobacco material.

The first sheet may be a textured sheet of tobacco material that is textured in a different way to the second sheet of tobacco material. For example, the first sheet may be crimped and the second sheet may be perforated.

Both first and second sheets may be crimped sheets that are morphologically different from each other. For example, the second sheet may be crimped with a different number of crimps per unit width of sheet compared to the first sheet.

By gathering together at least two sheets of tobacco material having different textures the physical properties of the rod may be controlled. For example, the density of the rod may be controlled by gathering at least two sheets of tobacco material that are differently textured.

As used herein, the term 'crimped sheet' is intended to be synonymous with the term 'creped sheet' and denotes a sheet having a plurality of substantially parallel ridges or corrugations. Preferably, a crimped sheet of tobacco material, for example a crimped sheet of homogenised tobacco material, has a plurality of ridges or corrugations substantially parallel to the cylindrical axis of the rod. This advantageously facilitates gathering of the crimped sheet of tobacco material to form the rod. However, it will be appreciated that crimped sheets of tobacco material for use in rods as described herein may alternatively or in addition have a plurality of substantially parallel ridges or corrugations disposed at an acute or obtuse angle to the cylindrical axis of the rod.

In certain embodiments, sheets of material for use in rods as described herein may be substantially evenly textured over substantially their entire surface. For example, crimped sheets of material for use in rods as described herein may comprise a plurality of substantially parallel ridges or corrugations that are substantially evenly spaced-apart across the width of the sheet.

The at least two sheets that are gathered together to form the rod may have different physical dimensions. The sheets are effectively endless sheets, and are supplied as a web to the manufacturing process. The width and thickness of the sheets may be varied, however.

It may be desirable to gather together two sheets each having a different thickness or each having a different width. This may alter the physical properties of the rod. This may facilitate the formation of a blended tobacco rod from sheets of tobacco material of different chemical composition.

The first sheet may have a first thickness and the second sheet may have a second thickness that is a percentage or a fraction of the first thickness, for example a half or a third or a quarter of the first thickness.

The first sheet may have a first width and the second sheet may have a second width that is different to the first width.

The first sheet of the first tobacco material and the second sheet of the second tobacco material are disposed in overlapping relationship prior to being gathered together. The sheets may have the same width and thickness. The sheets may have different thicknesses. The sheets may have different widths. The sheets may be differently textured.

Where it is desired that the first sheet and the second sheet are both textured, the sheets may be simultaneously textured prior to being gathered. For example, the sheets may be brought into overlapping relationship and passed through a texturing means, such as a pair of crimping rollers.

Alternatively, each sheet may be separately textured and then subsequently brought together to be gathered into a rod. For example, where the two sheets have a different thickness, it may be desirable to crimp the first sheet in a different way to the second sheet.

A rod may comprise one or more additional sheets of material gathered together with the first and second sheets to form the rod. Any additional sheet or sheets may be textured, for example crimped, prior to being gathered. Any additional sheet or sheets may comprise additional aerosol-forming materials, such as one or more additional sheets of homogenised tobacco.

A rod as described herein may be used as an aerosol-forming substrate in an aerosol-generating article.

An aerosol generating article may be provided comprising a rod as described herein.

Rods as described herein may be used as rods of combustible smokable material in lit-end smoking articles. In one embodiment, rods as described herein may be used as rods of combustible smokable material in lit-end cigarettes comprising a rod of combustible smokable material and a filter downstream of the rod of combustible smokable material.

In one embodiment, rods as described herein may be particularly beneficial as a component of a heated aerosol-generating article, for example as an aerosol-forming substrate of a heated aerosol-generating article.

Heated aerosol-generating systems operate by heating an aerosol-forming substrate to generate an aerosol from the material of the substrate. The aerosol can then be inhaled by a consumer.

A number of aerosol-generating articles in which an aerosol-forming substrate is heated rather than combusted have been proposed in the art. Typically in heated aerosol-generating articles, an aerosol is generated by the transfer of heat from a heat source, for example a chemical, electrical or combustible heat source, to a physically separate aerosol-forming substrate, which may be located within, around or downstream of the heat source.

As used herein, the term 'aerosol-forming substrate' denotes a substrate consisting of or comprising an aerosol-forming material that is capable of releasing volatile compounds upon heating to generate an aerosol. A sheet of tobacco material is an aerosol-forming substrate for the purposes of this specification.

Rods as described herein are particularly suited for use as aerosol-forming substrates of heated aerosol-generating articles. Aerosol-forming substrates in heated aerosol-generating articles are typically significantly shorter in rod length than rods of combustible smokable material in conventional lit-end smoking articles. As noted above, loose ends, high weight standard deviations and non-uniform densities as exhibited by rods comprising shreds of tobacco material are particularly undesirable in rods of aerosol-generating material having a short rod length. Use of short rods as described herein as aerosol-generating substrates in heated aerosol-generating articles advantageously minimises or avoids one or more of the disadvantages associated with the use of short rods comprising shreds of tobacco material previously discussed above.

In one embodiment, rods as described herein may be used as aerosol-forming substrates in heated aerosol-generating articles comprising a combustible heat source and an aerosol-generating substrate downstream of the combustible heat source.

For example, rods as described herein may be used as aerosol-generating substrates in heated aerosol-generating articles of the type disclosed in WO-A-2009/022232, which comprise a combustible carbon-based heat source, an aerosol-generating substrate downstream of the combustible heat source, and a heat-conducting element around and in contact with a rear portion of the combustible carbon-based heat source and an adjacent front portion of the aerosol-generating substrate. However, it will be appreciated that rods as described herein may also be used as aerosol-generating substrates in heated aerosol-generating articles comprising combustible heat sources having other constructions.

In another embodiment, rods as described herein may be used as aerosol-generating substrates in heated aerosol-generating articles for use in electrically-operated aerosol-generating systems in which the aerosol-generating substrate of the heated aerosol-generating article is heated by an electrical heat source.

For example, rods as described herein may be used as aerosol-generating substrates in heated aerosol-generating articles of the type disclosed in EP-A-0 822 670.

A system may be provided comprising an electrically-operated aerosol-generating apparatus and an aerosol-generating article for use with the apparatus. The aerosol-generating article comprises a rod or an aerosol-forming substrate as described herein.

The insertion and removal of heated aerosol-generating articles from an electrically-operated aerosol-generating system, for example an electrically-heated aerosol-generating system, where those articles include a rod comprising shreds of tobacco material, tends to dislodge shreds of tobacco material from the rod. This can disadvantageously result in the need for more frequent cleaning of the electrical heat source and other parts of the electrically-operated aerosol-generating system in order to remove the dislodged shreds.

In contrast, insertion and removal of heated aerosol-generating articles including an aerosol-generating substrate comprising a rod as described herein is less likely to result in dislodgement of tobacco material.

A filter for an aerosol-generating article may be provided, wherein the filter comprises a rod as described herein. Rods may be used in filters for both lit-end aerosol-generating articles, such as conventional smoking articles, and heated aerosol-generating articles. Rods as described herein may used in filters comprising a single filter segment. Rods as described herein may also be used in multi-component filters comprising two or more filter segments.

Filters comprising tobacco-containing filter segments are known in the art. For example, EP-A-1 889 550 discloses a multi-component filter for a smoking article comprising: a mouth end segment; a first flavour release segment comprising tobacco or other plant leaf upstream of the mouth end segment; and a second flavour release segment comprising filtration material and a flavourant upstream of the first flavour release segment. The resistance to draw of the second flavour release segment is greater than the resistance to draw of the first flavour release segment and the resistance to draw of the second flavour release segment is greater than the resistance to draw of mouth end segment.

In certain embodiments, rods as described herein may be used as tobacco-containing filter segments in single or multi-component filters. For example, rods as described herein may be used as a first flavor release segment in multi-component filters of the type disclosed in EP-A-1 889 550.

Filters comprising rods as described herein may further comprise one or more filtration materials for the removal of particulate components, gaseous components or a combination thereof. Suitable filtration materials are known in the art and include, but are not limited to: fibrous filtration materials such as, for example, cellulose acetate tow and paper; adsorbents such as, for example, activated alumina, zeolites, molecular sieves and silica gel; biodegradable polymers including, for example, polylatic acid (PLA), Mater-Bi®, and bioplastics; and combinations thereof.

Alternatively or in addition, filters comprising rods as described herein may further comprise one or more smoke or aerosol-modifying agents. Suitable smoke and aerosol-modifying agents are known in the art and include, but are not limited to: flavourants such as, for example, menthol.

Preferably, rods according to the specification are of substantially uniform cross-section.

Rods according to the specification may be produced having different dimensions depending upon their intended use.

For example, rods according to the specification may have a diameter of between about 5 mm and about 10 mm depending upon their intended use.

For example, rods according to the specification may have a rod length of between about 5 mm and about 150 mm depending upon their intended use.

In preferred embodiments, rods according to the specification for use as aerosol-forming substrates in heated aerosol-generating articles may have a rod length of between about 5 mm and about 20 mm or about 30 mm.

In further embodiments, rods according to the specification for use in filters for conventional lit-end smoking articles and heated aerosol-generating articles may have a rod length of between about 5 mm and about 30 mm.

Rods according to the specification of a desired unit rod length may be produced by forming a rod of multiple unit rod length and then cutting or otherwise dividing the rod of multiple unit rod length into multiple rods of the desired unit rod length.

For example, rods having a rod length of about 15 mm for use as aerosol-forming substrates in heated aerosol-generating articles may be produced by forming a rod having a rod length of about 150 mm and then severing the elongate rod into ten rods having a rod length of about 15 mm.

Preferred embodiments comprise sheets of homogenised tobacco material. Sheets of homogenised tobacco material may be formed by agglomerating particulate tobacco obtained by grinding or otherwise comminuting one or both of tobacco leaf lamina and tobacco leaf stems. Alternatively, or in addition, sheets of homogenised tobacco material tobacco may comprise one or more of tobacco dust, tobacco fines and other particulate tobacco by-products formed during, for example, the treating, handling and shipping of tobacco. Where rods according to the specification are intended for use as aerosol-forming substrates in heated aerosol-generating articles, sheets of homogenised tobacco material used to form the rods preferably comprise particulate tobacco obtained by grinding or otherwise comminuting tobacco leaf lamina.

In certain embodiments, sheets of homogenised tobacco material may have a tobacco content of at least about 40% by weight on a dry weight basis or of at least about 50% by weight on a dry weight basis. In other embodiments, sheets of homogenised tobacco material may have a tobacco content of about 70% or more by weight on a dry weight basis. Where rods according to the specification are intended for use as aerosol-forming substrates in heated aerosol-generating articles, the use of sheets of homogenised tobacco material having high tobacco contents advantageously generates aerosols with enhanced tobacco flavour.

Sheets of homogenised tobacco material may comprise one or more intrinsic binders, that is tobacco endogenous binders, one or more extrinsic binders, that is tobacco exogenous binders, or a combination thereof to help agglomerate the particulate tobacco. Alternatively, or in addition, sheets of homogenised tobacco material may comprise other additives including, but not limited to, tobacco and non-tobacco fibres, aerosol-formers, humectants, plasticisers, flavourants, fillers, aqueous and non-aqueous solvents and combinations thereof.

Suitable extrinsic binders for inclusion in sheets of homogenised tobacco material for use in forming a rod as described herein are known in the art and include, but are not limited to: gums such as, for example, guar gum, xanthan gum, arabic gum and locust bean gum; cellulosic binders such as, for example, hydroxypropyl cellulose, carboxymethyl cellulose, hydroxyethyl cellulose, methyl cellulose and ethyl cellulose; polysaccharides such as, for example, starches, organic acids, such as alginic acid, conjugate base salts of organic acids, such as sodium-alginate, agar and pectins; and combinations thereof.

Suitable non-tobacco fibres for inclusion in sheets of homogenised tobacco material are known in the art and include, but are not limited to: cellulose fibers; soft-wood fibres; hard-wood fibres; jute fibres and combinations thereof. Prior to inclusion in sheets of homogenised tobacco material, non-tobacco fibres may be treated by suitable processes known in the art including, but not limited to: mechanical pulping; refining; chemical pulping; bleaching; sulfate pulping; and combinations thereof.

Sheets of homogenised tobacco material for use in forming rods as described herein should have sufficiently high tensile strength to survive being gathered to form rods. In certain embodiments non-tobacco fibres may be included in sheets of homogenised tobacco material in order to achieve an appropriate tensile strength.

For example, homogenised sheets of tobacco material for forming rods as described herein may comprise between about 1% and about 5% non-tobacco fibres by weight on a dry weight basis.

Suitable aerosol-formers and humectants for inclusion in sheets of homogenised tobacco material are known in the art and include, but are not limited to: polyhydric alcohols, such as triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate.

For example, where rods according to the specification are intended for use as aerosol-forming substrates in heated aerosol-generating articles, sheets of homogenised tobacco material for use in forming rods as described herein may have an aerosol former content of between about 5% and about 30% by weight on a dry weight basis. Rods intended for use in electrically-operated aerosol-generating system having a heating element may preferably include an aerosol former of greater than 5% to about 30%. For rods intended for use in electrically-operated aerosol-generating system having a heating element, the aerosol former may preferably be glycerine.

It will be appreciated that the composition of sheets of homogenised tobacco material may be designed to comply with regulatory requirements.

A number of reconstitution processes for producing sheets of homogenised tobacco materials are known in the art. These include, but are not limited to: paper-making processes of the type described in, for example, US-A-3,860,012; casting or 'cast leaf' processes of the type described in, for example, US-A-5,724,998; dough reconstitution processes of the type described in, for example, US-A-3,894,544; and extrusion processes of the type described in, for example, in GB-A-983,928. Typically, the densities of sheets of homogenised tobacco material produced by extrusion processes and dough reconstitution processes are greater than the densities of sheets of homogenised tobacco materials produced by casting processes.

Sheets of homogenised tobacco material for use in forming rods as described herein are preferably formed by a casting process of the type generally comprising casting a slurry comprising particulate tobacco and one or more binders onto a conveyor belt or other support surface, drying the cast slurry to form a sheet of homogenised tobacco material and removing the sheet of homogenised tobacco material from the support surface.

For example, in certain embodiments sheets of homogenised tobacco material may be formed from slurry comprising particulate tobacco, guar gum, cellulose fibres and glycerine by a casting process.

Sheets of homogenised tobacco material may be textured using suitable known machinery for texturing filter tow, paper and other materials.

For example, sheets of homogenised tobacco material for forming rods as described herein may be crimped using a crimping unit of the type described in CH-A-691156, which comprises a pair of rotatable crimping rollers. However, it will be appreciated that sheets of homogenised tobacco material may be textured using other suitable machinery and processes that deform or perforate the sheets of homogenised tobacco material.

Rods according to the specification may be produced from sheets of homogenised tobacco material that have a range of different dimensions, depending upon the intended use of the rods. Sheets of homogeneous tobacco material should be of sufficient width to be gathered to form a rod as described herein.

Preferably, sheets of tobacco material for use in rods as described herein have a width of at least about 25 mm.

In certain embodiments sheets of tobacco material for use in rods as described herein may have a width of between about 25 mm and about 300 mm.

Preferably, sheets of tobacco material for use in rods as described herein have a combined thickness of at least about 50 µm to about 100 µm. Combined thickness refers to the total thickness of all sheets that make up the rod. For example, if the rod is formed from two sheets, then the combined thickness is the sum of the thickness of each individual sheet. Sheets of tobacco material for use in rods as described herein may have individual thicknesses of between about 25 µm to about 100 µm.

In certain embodiments, sheets of tobacco material for use in rods as described herein may have a thickness of between 10 µm and about 300 µm.

In certain embodiments, sheets of homogenised tobacco material for use in rods as described herein may have a grammage 100 g/m² and about 300 g/m².

Rods as described herein may comprise a gathered sheet of homogenised tobacco material circumscribed by a porous wrapper or a non-porous wrapper.

In certain embodiments, rods as described herein may comprise at least two sheets of homogenised tobacco material gathered together and circumscribed by a paper wrapper.

Suitable paper wrappers for use in specific embodiments of the invention are known in the art and include, but are not limited to: cigarette papers; and filter plug wraps.

In other embodiments, rods as described herein may comprise a non-paper wrapper.

Suitable non-paper wrappers for use in forming rods as described herein are known in the art and include, but are not limited to: homogenised tobacco materials.

Rods as described herein may be produced using conventional cigarette making and cigarette filter making machinery, adapted to allow for the gathering of two or more sheets simultaneously.

For example, rods comprising first and second crimped sheets of homogeneous tobacco material may be produced using an adaptation of machinery for forming filter rods comprising a gathered crimped sheet of paper of the type described in CH-A-691156. The machinery could be adapted to allow two sheets of material to be crimped and gathered.

A method of forming a rod may be provided comprising the steps of: providing a first continuous sheet of tobacco material, providing a second continuous sheet of tobacco material; wherein both the first tobacco material and the second tobacco material are homogenised tobacco material, the first tobacco material being chemically different to the second tobacco material, the first homogenised tobacco material comprising aerosol-forming compounds that form an aerosol at a lower temperature than aerosol-forming compounds of the second homogenised tobacco material; disposing the first continuous sheet of the first tobacco material and the second continuous sheet of the second tobacco material in overlapping relationship; simultaneously gathering the first and second continuous sheets of tobacco material transversely relative to the longitudinal axes thereof; circumscribing the gathered sheets of tobacco material with a wrapper to form a continuous rod, and severing the continuous rod into a plurality of discrete rods.

The method may further comprise texturing the first continuous sheet. For example, the method may comprise crimping, embossing, perforating or otherwise texturing the first continuous sheet prior to gathering the first continuous sheet together with the second continuous sheet.

Preferably, the method further comprises crimping the first continuous sheet.

Both first and second continuous sheets may be textured, for example crimped. Both first and second sheets of tobacco material may be crimped prior to being gathered.

Specific embodiments will be further described, by way of example only, with reference to the accompanying drawings in which:
Figure 1 shows a schematic cross-section of apparatus for forming a rod according to a specific embodiment;
Figure 2 shows a schematic cross-section of apparatus for forming a rod according to a specific embodiment;
Figures 3 to 8 illustrate cross-sectional views of sheets of tobacco material immediately prior to entering a gathering means to be gathered into a rod;
Figures 9 and 10 illustrate embodiments of aerosol-generating devices that incorporate rods formed as described herein; and
Figure 11 illustrates an aerosol-generating system comprising an electrically-operated aerosol-generating device and an aerosol-generating article as illustrated in Figure 9.

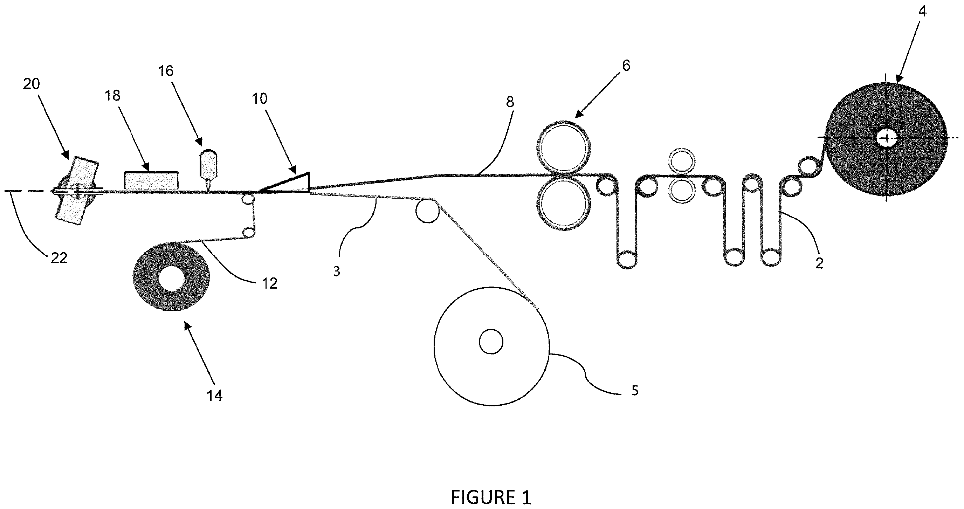

The apparatus shown in Figure 1 generally comprises: first supply means for providing a first continuous sheet of homogenised tobacco material; second supply means for providing a second continuous sheet of homogenised material; crimping means for crimping the first continuous sheet of homogenised tobacco material; rod forming means for gathering both the first and second continuous sheets of homogenised tobacco material together and circumscribing the gathered material with a wrapper to form a continuous rod; and cutting means for severing the continuous rod into a plurality of discrete rods. The apparatus also comprises transport means for transporting at least the first continuous sheet of homogenised tobacco material downstream through the apparatus from the supply means to the rod forming means via the crimping means.

As shown in Figure 1, the first supply means for providing a first continuous sheet of homogenised tobacco material comprises a continuous sheet of homogenised tobacco material 2 mounted on a first bobbin 4. The second supply means for providing a second continuous sheet of homogenised tobacco material comprises a continuous sheet of homogenised tobacco material 3 mounted on a second bobbin 5.The crimping means comprises a pair of rotatable crimping rollers 6. In use, the first continuous sheet of homogenised tobacco material 2 is drawn from the first bobbin 4 and transported downstream to the pair of crimping rollers 6 by the transport mechanism via a series of guide and tensioning rollers. As the continuous sheet of homogenised tobacco material 2 is fed between the pair of crimping rollers 6, the crimping rollers engage and crimp the first continuous sheet of homogenised tobacco material 2 to form a first continuous crimped sheet of homogenised tobacco material 8 having a plurality of spaced-apart ridges or corrugations substantially parallel to the longitudinal axis of the sheet of homogenised tobacco material through the apparatus.

The first continuous crimped sheet of homogenised tobacco material 8 is transported downstream from the pair of crimping rollers 6 towards the rod forming means. The second continuous sheet of homogenised tobacco material 3 is transported from the second bobbin 5 towards the rod forming means. Both the second continuous sheet of homogenised tobacco material 3 and the first continuous sheet of crimped homogenised tobacco material 8 are simultaneously fed through a converging funnel or horn 10. The converging funnel 10 gathers the continuous sheets of material 8, 3 transversely relative to their longitudinal axes. The continuous sheets of material 8,3 assume a substantially cylindrical configuration as they pass through the converging funnel 10.

Upon exiting the converging funnel 10, the gathered sheets of homogenised tobacco material are wrapped in a continuous sheet of wrapping material 12. The continuous sheet of wrapping material is fed from a bobbin 14 and enveloped around the gathered sheets of material by an endless belt conveyor or garniture. As shown in Figure 1, the rod forming means comprises an adhesive application means 16 that applies adhesive to one of the longitudinal edges of the continuous sheet of wrapping material, so that when the opposed longitudinal edges of the continuous sheet of wrapping material are brought into contact they adhere to one other to form a continuous rod.

The rod forming means further comprises a drying means 18 downstream of the adhesive application means 16, which in use dries the adhesive applied to the seam of the continuous rod as the continuous rod is transported downstream from the rod forming means to the cutting means.

The cutting means comprises a rotary cutter 20 that severs the continuous rod into a plurality of discrete rods of unit rod length or multiple unit rod length.

As the two continuous sheets of material are fed into the converging funnel while overlaid, one sheet on top of the other, the rod has an even distribution of the first sheet of tobacco material and the second sheet of tobacco material.

Although the second continuous sheet of homogenised tobacco material is depicted as being gathered into a rod without being crimped, it is clear that this second continuous sheet may pass through a set of crimping rollers to be crimped prior to being gathered into a rod.

To manufacture a rod comprising a sheet of a first tobacco material and a continuous element of a second tobacco material having a different chemical composition to the first tobacco material, the second sheet of homogenised tobacco material on the second bobbin (as illustrated in Figure 1) could be replaced with a bobbin holding a suitable continuous element. Such an element may take the form of a ribbon, strip, tape or thread. Operation of the process would otherwise be as described above.

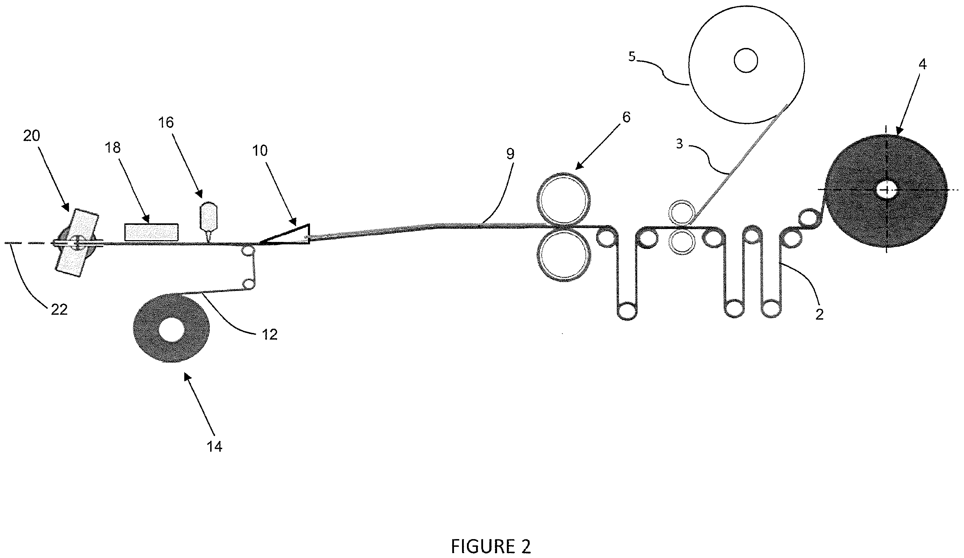

In an alternative configuration illustrated in Figure 2, a second continuous sheet of homogenised tobacco material 3 is positioned in overlapping relationship with a first continuous sheet of homogenised tobacco material 2 upstream of a pair of crimping rollers 6. The apparatus is otherwise substantially as described above in relation to Figure 1.

Both continuous sheets of material 2,3 pass through the crimping rollers 6 in overlapping relationship and are simultaneously crimped. A crimped pair of continuous sheets 9 passes out of the crimping rollers 6 and downstream into the converging funnel 10 to be formed into a rod.

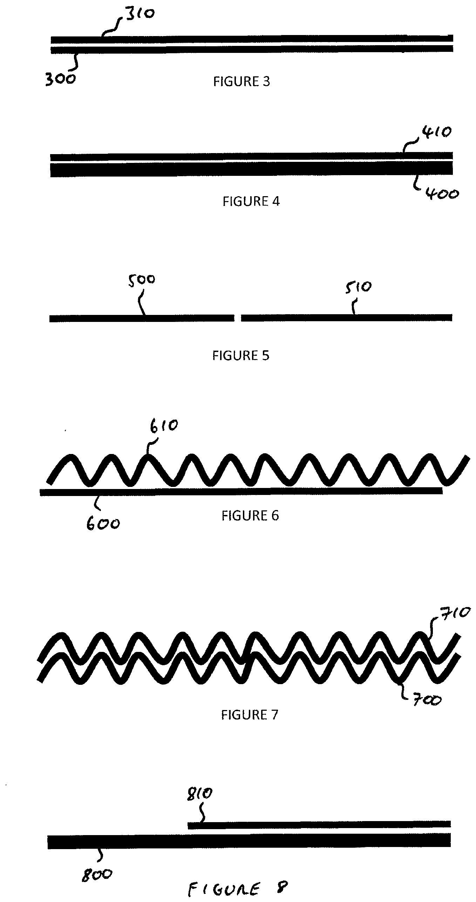

Figures 3 to 8 provide schematic cross-sectional illustrations of different possible arrangements of two sheets of tobacco material immediately prior to entering into a converging funnel or horn in order to be gathered into a rod. All of these Figures illustrate the relative thickness, width, and cross-sectional shape of each sheet of tobacco. The length dimension of each of the sheets in these schematic illustrations extends into the plane of the paper.

Figure 3 illustrates an arrangement in which a first sheet of tobacco material 300 is overlaid with a second sheet of tobacco material 310 of the same dimensions prior to both sheets entering a gathering device to be gathered into a rod. While the physical dimensions of each sheet are the same, the chemical composition of each sheet is different.

Figure 4 illustrates an arrangement in which a first sheet of tobacco material 400 is overlaid with a second sheet of tobacco material 410 that has a smaller thickness dimension compared to the first tobacco sheet 400. While the physical dimensions of each sheet differ, in that one sheet is thinner than the other, the chemical composition of each sheet will also differ.

Figure 5 illustrates an arrangement in which a first sheet of tobacco material 500 is positioned adjacent to a second sheet of tobacco material 510 of the same dimensions prior to both sheets entering a gathering device to be are gathered into a rod. While the physical dimensions of each sheet are the same, the chemical composition of each sheet is different.

Figure 6 illustrates an arrangement in which a first sheet of tobacco material 600 is overlaid with a second sheet of tobacco material 610 prior to both sheets entering a gathering device to be gathered into a rod. The second sheet has been crimped. The composition of the two sheets is different.

Figure 7 illustrates an arrangement in which a first crimped sheet of tobacco material 700 is overlaid with a second crimped sheet of tobacco material 710 prior to both sheets entering a gathering device to be gathered into a rod. While the physical dimensions of each sheet are the same, the chemical composition of each sheet is different.

Figure 8 illustrates an arrangement in which a first sheet of tobacco material 800 is overlaid with a second sheet of tobacco material 810 prior to both sheets entering a gathering device to be gathered into a rod. The second sheet 810 has a lesser width and thickness compared with the first sheet 800.

The skilled person will appreciate that many more arrangements of two or more sheets of tobacco material may be possible.

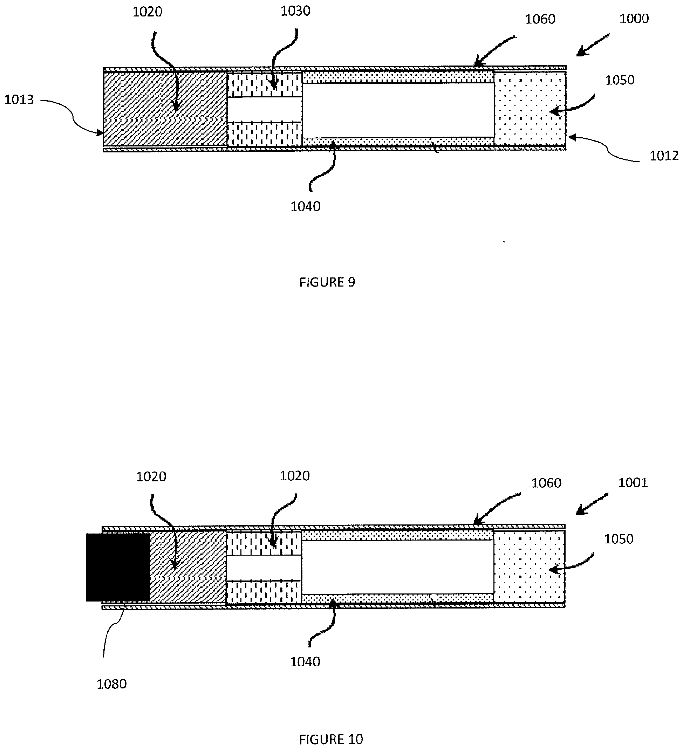

Figure 9 illustrates an embodiment of an aerosol-generating article 1000 comprising a rod as described herein. The article 1000 comprises four elements; an aerosol-forming substrate 1020, a hollow cellulose acetate tube 1030, a spacer element 1040, and a mouthpiece filter 1050. These four elements are arranged sequentially and in coaxial alignment and are assembled by a cigarette paper 1060 to form the aerosol-generating article 1000. The article 1000 has a mouth-end 1012, which a user inserts into his or her mouth during use, and a distal end 1013 located at the opposite end of the article to the mouth end 1012. The embodiment of an aerosol-generating article illustrated in Figure 9 is particularly suitable for use with an electrically-operated aerosol-generating device comprising a heater for heating the aerosol-forming substrate.

When assembled, the article 1000 is about 45 millimetres in length and has an outer diameter of about 7.2 millimetres and an inner diameter of about 6.9 millimetres.

The aerosol-forming substrate 1020 comprises a rod formed from a first sheet of crimped cast-leaf tobacco and a second sheet of crimped cast-leaf tobacco wrapped in a filter paper (not shown) to form a plug. Both sheets of cast-leaf tobacco include additives, including glycerine as an aerosol-forming additive. The first sheet of cast-leaf tobacco and the second sheet of cast-leaf comprise different blends of tobacco and therefore differ chemically.

An aerosol-generating article 1000 as illustrated in Figure 9 is designed to engage with an aerosol-generating device in order to be consumed. Such an aerosol-generating device includes means for heating the aerosol-forming substrate 1020 to a sufficient temperature to form an aerosol. Typically, the aerosol-generating device may comprise a heating element that surrounds the aerosol-generating article 1000 adjacent to the aerosol-forming substrate 1020, or a heating element that is inserted into the aerosol-forming substrate 1020.

Once engaged with an aerosol-generating device, a user draws on the mouth-end 1012 of the smoking article 1000 and the aerosol-forming substrate 1020 is heated to a temperature of about 375 degrees Celsius. At this temperature, volatile compounds are evolved from the two different sheets of cast-leaf tobacco of the aerosol-forming substrate 1020. These compounds condense to form an aerosol. The aerosol is drawn through the filter 1050 and into the user's mouth.

Figure 10 illustrates a second embodiment of an aerosol-generating article1001. While the article of Figure 9 is intended to be consumed in conjunction with an aerosol-generating device, the article of Figure 10 comprises a combustible heat source 1080 that may be ignited and transfer heat to the aerosol-forming substrate 1020 to form an inhalable aerosol. The combustible heat source 80 is a charcoal element that is assembled in proximity to the aerosol-forming substrate at a distal end 13 of the rod 11. Elements that are essentially the same as elements in Figure 9 have been given the same numbering.

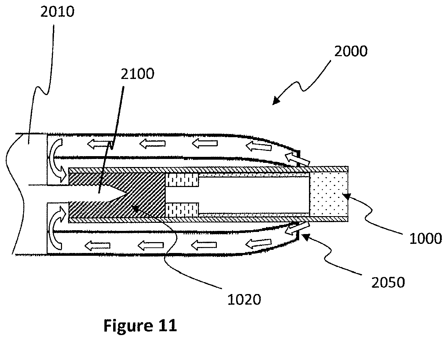

Figure 11 illustrates a portion of an electrically-operated aerosol-generating system 2000 that utilises a heating blade 2100 to heat an aerosol-generating substrate 1020 of an aerosol-generating article 1000. The heating blade is mounted within an aerosol article receiving chamber of an electrically-operated aerosol-generating device 2010. The aerosol-generating device defines a plurality of air holes 2050 for allowing air to flow to the aerosol-generating article 1000. Air flow is indicated by arrows on Figure 11. The aerosol-generating device comprises a power supply and electronics, which are not illustrated in Figure 11. The aerosol-generating article 1000 of Figure 11 is as described in relation to Figure 9.

### Example 1

Rods according to a specific embodiment of the invention comprise a first crimped sheet of homogenised tobacco material gathered together with a second crimped sheet of homogenised tobacco material, and circumscribed by a paper wrapper. The rods have a rod length of 12 mm and diameters of between 6.9 mm and 7.2 mm. The rods were produced at rates of between 20 m/min and 25 m/min using apparatus of the type shown in Figure 1.

The first continuous sheet of homogenised tobacco material was produced by a casting process, the sheets having a width of between 110 mm and 134 mm, a thickness of 120 µm to 260 µm, a grammage of between 167 g/m² and 201 g/m² and a moisture content of between 5% and 12%. The first continuous sheet comprises about 10% by weight of glycerine.

The second continuous sheet of homogenised tobacco material was produced by a casting process, the sheets having a width of between 110 mm and 134 mm, a thickness of 120 µm to 260 µm, a grammage of between 167 g/m² and 201 g/m² and a moisture content of between 5% and 12%. The second continuous sheet comprises about 10% by weight of propylene glycol.

The first continuous sheet of homogenised tobacco and the second continuous sheet of homogenised tobacco were crimped by being arranged in overlapping relationship and passing through a crimping roller together prior to being gathered into a rod.

The exemplary embodiments and example described above are not limiting. In view of the above-discussed exemplary embodiments, other embodiments consistent with the above exemplary embodiment will now be apparent to one of ordinary skill in the art.

## Claims

1. A rod comprising a first sheet of a first tobacco material (2) and a second sheet formed of a second tobacco material (3), wherein the second sheet is gathered together with the first sheet of the first tobacco material (2) and circumscribed by a wrapper (12), wherein the first sheet of the first tobacco material (2) and the second sheet of the second tobacco material (3) have been disposed in overlapping relationship prior to being gathered together, the first tobacco material (2) and the second tobacco material (3) being homogenised tobacco material, the second tobacco material (3) being chemically different to the first tobacco material (2), wherein the first tobacco material (2) comprises aerosol-forming compounds that form an aerosol at a lower temperature than aerosol-forming compounds of the second tobacco material (3).

2. A rod according to claim 1 in which both the first and second tobacco materials comprise an aerosol-former, the proportion by weight of aerosol-former in the first tobacco material being different to the proportion by weight of aerosol-former in the second tobacco material.

3. A rod according to claim 1 or 2 in which the first tobacco material comprises a first aerosol-former and the second tobacco material comprises a second aerosol-former different to the first aerosol-former.

4. A rod according any preceding claim in which at least one of the first and second sheets of tobacco material is crimped.

5. A rod according to any preceding claim comprising a further continuous element of a third tobacco material.

6. An aerosol-generating article, for example a smoking article, comprising a rod according to any of claims 1 to 5.

7. An aerosol-forming substrate for a heated aerosol-generating article, comprising a rod according to any of claims 1 to 5.

8. An aerosol-generating article comprising a combustible heat source and an aerosol-forming substrate according to claim 7 located downstream of the combustible heat source.

9. An aerosol-generating article for use in an electrically-operated aerosol-generating system comprising an aerosol-forming substrate according to claim 7.

10. A system comprising an electrically-operated aerosol-generating apparatus and an aerosol-generating article for use with the apparatus, the aerosol-generating article comprising an aerosol-forming substrate according to claim 7.

11. A method of forming a rod comprising the steps of:
providing a first continuous sheet of a first tobacco material (2);
providing a further continuous sheet of a second tobacco material; wherein both the first tobacco material (2) and the second tobacco material (3) are homogenised tobacco material, the first tobacco material (2) being chemically different to the second tobacco material (3), the first homogenised tobacco material (2) comprising aerosol-forming compounds that form an aerosol at a lower temperature than aerosol-forming compounds of the second homogenised tobacco material (3);
disposing the first continuous sheet of the first tobacco material (2) and the second continuous sheet of the second tobacco material (3) in overlapping relationship;
simultaneously gathering together the first continuous sheet and the further continuous sheet;
circumscribing the gathered first and second tobacco continuous sheets with a wrapper (12) to form a continuous rod; and
severing the continuous rod into a plurality of discrete rods.

12. A method according to claim 11 in which at least the first continuous sheet of the first tobacco material is crimped prior to being gathered.

13. A method according to claim 11 or 12 in which both the first sheet and the second sheet are crimped prior to being gathered.

## Patentansprüche

1. Stock, der ein erstes Flächengebilde aus einem ersten Tabakmaterial (2) und ein zweites, aus einem zweiten Tabakmaterial (3) gebildetes Flächengebilde aufweist, wobei das zweite Flächengebilde mit dem ersten Flächengebilde aus dem ersten Tabakmaterial (2) zusammengefasst und durch eine Umhüllung (12) abgegrenzt ist, wobei das erste Flächengebilde aus dem erste Tabakmaterial (2) und das zweite Flächengebilde aus dem zweiten Tabakmaterial (3) vor dem Zusammenfassen in überlappender Beziehung angeordnet sind, wobei das erste Tabakmaterial (2) und das zweite Tabakmaterial (3) homogenisiertes Tabakmaterial ist, wobei sich das zweite Tabakmaterial (3) chemisch vom ersten Tabakmaterial (2) unterscheidet, wobei das erste Tabakmaterial (2) aerosolbildende Verbindungen aufweist, die ein Aerosol bei einer niedrigeren Temperatur als aerosolbildende Verbindungen des zweiten Tabakmaterials (3) bilden.

2. Stock nach Anspruch 1, wobei sowohl das erste als auch das zweite Tabakmaterial einen Aerosolbildner aufweisen, wobei sich der Gewichtsanteil des Aerosolbildners in dem ersten Tabakmaterial vom Gewichtsanteil des Aerosolbildners in dem zweiten Tabakmaterial unterscheidet.

3. Stock nach Anspruch 1 oder 2, wobei das erste Tabakmaterial einen ersten Aerosolbildner aufweist und das zweite Tabakmaterial einen zweiten Aerosolbildner aufweist, der sich von dem ersten Aerosolbildner unterscheidet.

4. Stock nach einem der vorstehenden Ansprüche, wobei zumindest eines von dem ersten und dem zweiten Flächengebilde gewellt ist.

5. Stock nach einem der vorstehenden Ansprüche, der ein weiteres kontinuierliches Element eines dritten Tabakmaterials aufweist.

6. Aerosolerzeugender Artikel, beispielsweise ein Raucherartikel, der einen Stock nach einem der Ansprüche 1 bis 5 aufweist.

7. Aerosolbildendes Substrat für einen erwärmten aerosolerzeugenden Artikel, der einen Stock nach einem der Ansprüche 1 bis 5 aufweist.

8. Aerosolerzeugender Artikel, der eine brennbare Wärmequelle und ein aerosolbildendes Substrat nach Anspruch 7 aufweist, das sich nachgeschaltet der brennbaren Wärmequelle befindet.

9. Aerosolerzeugender Artikel zur Verwendung bei einem elektrisch betriebenen Aerosolerzeugungssystem, das ein aerosolbildendes Substrat nach Anspruch 7 aufweist.

10. System, das eine elektrisch betriebene aerosolerzeugende Vorrichtung und einen aerosolerzeugenden Artikel zur Verwendung mit der Vorrichtung aufweist, wobei der aerosolerzeugende Artikel ein aerosolbildendes Substrat nach Anspruch 7 aufweist.

11. Verfahren zum Ausbilden eines Stocks, umfassend die folgenden Schritte:
Bereitstellen eines ersten kontinuierlichen Flächengebildes aus einem ersten Tabakmaterial (2);
Bereitstellen eines weiteren kontinuierlichen Flächengebildes aus einem zweiten Tabakmaterial; wobei sowohl das erste Tabakmaterial (2) als auch das zweite Tabakmaterial (3) homogenisiertes Tabakmaterial ist, wobei sich das erste Tabakmaterial (2) chemisch vom zweiten Tabakmaterial unterscheidet, wobei das erste homogenisierte Tabakmaterial (2) aerosolbildende Verbindungen aufweist, die ein Aerosol bei einer niedrigeren Temperatur als aerosolbildende Verbindungen des zweiten homogenisierten Tabakmaterials (3) bilden;
Anordnen des ersten kontinuierlichen Flächengebildes aus dem ersten Tabakmaterial (2) und des zweiten kontinuierlichen Flächengebildes aus dem zweiten Tabakmaterial (3) in überlappender Beziehung;
gleichzeitiges Zusammenfassen des ersten kontinuierlichen Flächengebildes und des weiteren kontinuierlichen Flächengebildes;
Abgrenzen der zusammengefassten ersten und zweiten kontinuierlichen Tabakflächengebilde mit einer Umhüllung (12), um einen kontinuierlichen Stock zu bilden; und
Trennen des kontinuierlichen Stocks in eine Vielzahl von diskreten Stöcken.

12. Verfahren nach Anspruch 11, wobei zumindest das erste kontinuierliche Flächengebilde aus dem ersten Tabakmaterials gewellt ist, bevor es zusammengefasst wird.

13. Verfahren nach Anspruch 11 oder 12, wobei sowohl das erste als auch das zweite Flächengebilde gewellt sind, bevor sie zusammengefasst werden.

## Revendications

1. Tige comprenant une première feuille d'un premier matériau de tabac (2) et une seconde feuille formée d'un second matériau de tabac (3), dans laquelle la seconde feuille est assemblée avec la première feuille du premier matériau de tabac (2) et entourée par une enveloppe (12), dans laquelle la première feuille du premier matériau de tabac (2) et la seconde feuille du second matériau de tabac (3) ont été disposées en relation de chevauchement avant d'être rassemblées, le premier matériau de tabac (2) et le second matériau de tabac (3) étant un matériau de tabac homogénéisé, le second matériau de tabac (3) étant chimiquement différent du premier matériau de tabac (2), dans lequel le premier matériau de tabac (2) comprend des composés formant aérosol qui forment un aérosol à une température inférieure à celle des composés formant un aérosol du second matériau de tabac (3).

2. Tige selon la revendication 1, dans laquelle les premier et second matériaux de tabac comprennent tous deux un formateur d'aérosol, la proportion en poids du formateur d'aérosol dans le premier matériau de tabac étant différente de la proportion en poids du formateur d'aérosol dans le second matériau de tabac.

3. Tige selon la revendication 1 ou 2, dans laquelle le premier matériau de tabac comprend un premier générateur d'aérosol et le second matériau de tabac comprend un second générateur d'aérosol différent du premier générateur d'aérosol.

4. Tige selon une quelconque revendication précédente, dans laquelle au moins une parmi la première feuille et la seconde feuille de tabac est crêpée.

5. Tige selon l'une quelconque des revendications précédentes, comprenant un élément continu supplémentaire d'un troisième matériau de tabac.

6. Article de génération d'aérosol, par exemple un article à fumer, comprenant une tige selon l'une quelconque des revendications 1 à 5.

7. Substrat formant aérosol pour un article de génération d'aérosol chauffé comprenant une tige selon l'une quelconque des revendications 1 à 5.

8. Article de génération d'aérosol chauffé comprenant une source de chaleur combustible et un substrat formant aérosol selon la revendication 7 situé en aval de la source de chaleur combustible.

9. Article de génération d'aérosol chauffé pour une utilisation dans un système de génération d'aérosol chauffé électriquement comprenant un substrat formant aérosol selon la revendication 7.

10. Système comprenant un appareil de génération d'aérosol à fonctionnement électrique et un article de génération d'aérosol pour l'utilisation avec l'appareil, l'article de génération d'aérosol comprenant un substrat formant aérosol selon la revendication 7.

11. Procédé de formation d'une tige comprenant les étapes suivantes :
la fourniture d'une première feuille continue d'un premier matériau de tabac (2) ;
la fourniture d'une autre feuille continue d'un deuxième matériau de tabac ; dans lequel le premier matériau de tabac (2) et le second matériau de tabac (3) sont tous deux des matériaux de tabac homogénéisés, le premier matériau de tabac (2) étant chimiquement différent du second matériau de tabac, le premier matériau de tabac homogénéisé (2) comprenant des composés formant aérosol qui forment un aérosol à une température inférieure à celle des composés formant un aérosol du second matériau de tabac homogénéisé (3) ;
la disposition de la première feuille continue du premier matériau de tabac (2) et de la seconde feuille continue du second matériau de tabac (3) en relation de chevauchement ;
le rassemblement simultané de la première feuille continue et de la feuille continue supplémentaire ;
la circonstance des première et seconde feuilles continues de tabac assemblées avec une enveloppe (12) pour former une tige continue ; et
la séparation de la tige continue en une pluralité de tiges distinctes.

12. Procédé selon la revendication 11, dans lequel l'au moins la première feuille continue du premier matériau de tabac est crêpée avant d'être assemblée.

13. Procédé selon l'une quelconque des revendications 11 ou 12, dans lequel à la fois la première et la deuxième feuille sont crêpées avant d'être assemblées.
